# EUROPEAN PATENT SPECIFICATION

(11) **EP 3 366 191 B1**
(45) Date of publication and mention of the grant of the patent: **17.11.2021**
(21) Application number: 18158902.9
(22) Date of filing: 27.02.2018
(51) Int. Cl.: A61B 1/00

(54) **ENDOSCOPE SYSTEM**
ENDOSKOPSYSTEM
SYSTÈME D'ENDOSCOPE

(30) Priority: 28.02.2017 JP 2017035840
(43) Date of publication of application: 29.08.2018
(73) Proprietor: FUJIFILM Corporation, Tokyo 106-8620 (JP)
(72) Inventor: KUBO, Masahiro, Kanagawa, Kanagawa 258-8538 (JP); OZAWA, Satoshi, Kanagawa, Kanagawa 258-8538 (JP); TATSUTA, Takeichi, Kanagawa, Kanagawa 258-8538 (JP); KURAMOTO, Masayuki, Kanagawa, Kanagawa 258-8538 (JP); SUGIZAKI, Makoto, Kanagawa, Kanagawa 258-8538 (JP)
(74) Representative: Klunker IP Patentanwälte PartG mbB

(56) References cited:
- EP-A1- 2 638 843
- JP-A- 2012 070 938
- US-A1- 2009 244 485
- US-A1- 2011 228 064
- US-A1- 2015 057 498
- US-A1- 2016 210 731

## Description

### BACKGROUND OF THE INVENTION

### 1. Field of the Invention

The present invention relates to an endoscope system and a method of operating the same that are used for screening to be performed in a medical examination center or a clinic.

### 2. Description of the Related Art

In the medical field, diagnosis using an endoscope system including a light source device, an endoscope, and a processor device has been performed widely. The endoscope system irradiates an observation object via the endoscope with illumination light from the light source device, and the processor device produces an image of the observation object on the basis of an image signal obtained by imaging the observation object under illumination with the illumination light. A doctor can perform diagnosis by displaying this image on a monitor while viewing the image on the monitor.

In recent years, the number of facilities that execute an endoscopic examination as a screening examination has been increasing in number, and a high-quality examination is required so that a correct diagnosis can be performed even in a case where a doctor who executes the endoscopic examination is not necessarily rich in experience. Thus, guidelines (manual) for imaging the inside of an alimentary canal thoroughly at an appropriate level are defined so that determination can be objectively performed even in a case where experience with the endoscope is not rich (for example, refer to "Stomach Endoscope Medical Examination Manual for Countermeasure Type Medical Examination; 2015 Edition (Pages 56 to 63)"). Additionally, thereafter, double-checking as to whether or not the captured image has been captured according to the guidelines is also performed.

In guidelines regarding the examination of the stomach, for example, the followings are defined.
- The number of images required is about 40 frames from the pharynx to the duodenum.
- The order (route) of observation is made constant.
- There is no point (blind spot) that is not observed (the entire circumference of a cardiac section, sensory root/posterior wall of premise section/lesser curvature section).
- The stomach is sufficiently inflated (constant-pressure air supply).
- Being imaged with suitable brightness.
- Mucous membrane is removed.
- Scenes including landmarks are imaged.

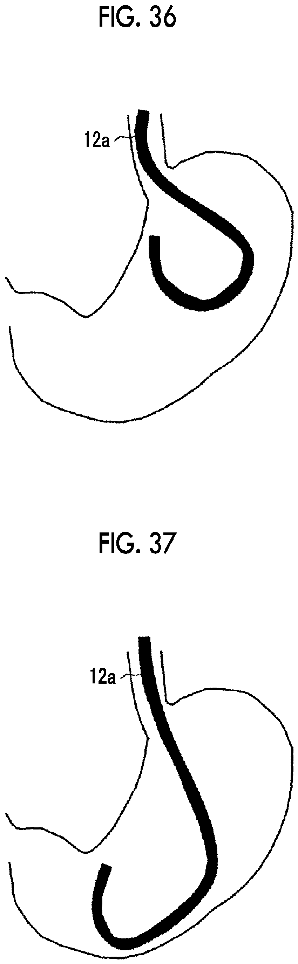

However, in order to perform imaging according to the guidelines, certain skills and experience are required for the operation of the endoscope. For example, as illustrated in Figs. 36 and 37, in a case where a site of a section directly below the cardia or a gastric angle, which is defined by the guidelines, is captured, a bending operation largely rotating a distal end part of the endoscope is required. Additionally, in the guideline, imaging of a lesion site hidden between the ribs is also required. In such a case, it is necessary to spread the ribs to confirm the presence/absence of the lesion site by blowing supply gas with suitable air pressure. In this way, it is not easy that less-experienced doctors perform the imaging or operation defined by the guidelines even in a case where the doctors are medical specialists, and an assist technique capable of performing imaging according to the guidelines is required.

In contrast, several techniques are disclosed as shown in JP2012-70938A, JP2012-70937A, and JP2012-70936A (JP2012-70938A and JP2012-70936A are corresponding to US2012/078045A1). In JP2012-70938A, in a case where the number of images defined by the guidelines does not reach a predetermined number, extracting required images from a moving image is performed. Additionally, in JP2012-70937A, the insertion length of an insertion part of an endoscope is detected, and required images are automatically captured according to the insertion length. Additionally, in JP2012-70936A, in a case where the number of images in a predetermined examination site does not coincide with a predetermined number of images, an alert is issued so that a user is notified of the failure to capture images.

EP-A-2 638 843 discloses an endoscope system, processor device thereof, and exposure control method. When the endoscope system is put into a special mode, first and second frame periods for performing imaging under first and second measurement light to measure an oxygen saturation level, a third frame period for performing imaging under normal light, and a fourth frame period for performing imaging under vessel detection light to detect blood vessels in specific depth are repeated. An oxygen saturation image, a normal image, and a vessel pattern image are produced and displayed in a tiled manner on a monitor in the form of moving images. When a freeze button is pressed during display of the moving images, the light intensity and exposure time to be used in the first to fourth frame periods of a still image recording process are calculated using an image that is captured immediately before pressing the freeze button. Still images are obtained with the calculated light intensity and exposure time.

### SUMMARY OF THE INVENTION

However, in the assist techniques of JP2012-70938A, JP2012-70937A, and JP2012-70936A alone, it is difficult to know whether or not imaging is performed according to the guidelines. For example, in JP2012-70938A, the images required for a guideline are extracted from the moving image, but images cannot be extracted in a case where no moving image is captured at a position defined by the guideline. Additionally, in JP2012-70937A, imaging is automatically performed according to the insertion length. However, since the insertion length may vary according to the patients' physique differences, a position where imaging is performed according to the insertion length may not coincide with a position defined by the guidelines. Additionally, in JP2012-70936A, even in a case where the alert is received and imaging is performed again, there is a case where less-experienced doctors cannot perform imaging according to the guidelines in a case where there is no certain assist for performing the imaging according to the guidelines.

An object of the present invention is to provide an endoscope system and a method of operating the same capable of assisting in capturing an image defined by guidelines.

According to one aspect, there is provided an endoscope system according to claim 1 of the appended claims.

An endoscope system of the present invention comprises a reference imaging condition storage unit that stores a reference imaging condition for obtaining a predetermined reference image for each examination region; an image acquisition unit that images the examination region to acquire an examination image in order to obtain a reference-equivalent image equivalent to the reference image; an at-examination imaging condition acquisition unit that acquires an at-examination imaging condition obtained at an acquisition timing of the examination image; a still image saving request signal output unit that outputs a still image saving request signal for requesting saving of a still image of the examination image in a case where the at-examination imaging condition satisfies the reference imaging condition; and a display unit that displays the examination image.

It is preferable that the endoscope system further comprises a reference image storage unit that stores a plurality of the reference images; and a reference image selection unit that selects a reference image corresponding to the examination image from the reference image storage unit, and the display unit displays the reference image selected by the reference image selection unit in a case where the still image saving request signal is output. It is preferable that the endoscope system further comprises a reference image storage unit that stores a plurality of the reference images; a reference image selection unit that selects a reference image corresponding to the examination image from the reference image storage unit; and a similarity
calculation unit that calculates a similarity between the reference image selected by the reference image selection unit and the examination image, and the display unit displays the similarity in a case where the still image saving request signal is output.

It is preferable that the endoscope system further comprises a still image storage control unit that performs a control of storing the still image of the examination image into a saving-image storage unit in a case where the still image saving request signal is output and the operation of a still image acquisition instruction unit is performed. It is preferable that, in a case where the still image of the examination image is obtained in a specific examination region, the display unit displays that the still image of the examination image in the specific examination region has been saved in the saving-image storage unit on an examination target map.

It is preferable that the display unit displays operation assist information on the operation of an endoscope in a case where the still image saving request signal is output and the still image of the examination image is not stored in a saving-image storage unit. It is preferable that the endoscope system further comprises a mode switching unit that performs switching to an automatic imaging mode in which the still image of the examination image is automatically acquired in a case where the at-examination imaging condition satisfies the reference imaging condition.

It is preferable that the reference imaging condition is at least one of endoscope positional information or endoscope operation information for obtaining the reference image, and the at-examination imaging condition is at least one of endoscope positional information or endoscope operation information for obtaining the examination image. It is preferable that at least one of a bending angle or rotational amount of an endoscope, a distance from an observation object, the endoscope operation information includes an insertion length of an insertion part of the endoscope, and a shape of the insertion part of the endoscope.

A method of operating an endoscope system of the present invention comprises a step of allowing an image acquisition unit to image an examination region to acquire an examination image in order to obtain a reference-equivalent image equivalent to a predetermined reference image for each examination region; a step of allowing an at-examination imaging condition acquisition unit to acquire an at-examination imaging condition obtained at an acquisition timing of the examination image; and a step of allowing a still image saving request signal output unit to output a still image saving request signal for requesting saving of a still image of the examination image in a case where the at-examination imaging condition satisfies a reference imaging condition for obtaining the reference image.

According to the present invention, it is possible to assist in capturing an image defined by guidelines.

### BRIEF DESCRIPTION OF THE DRAWINGS

Fig. 1 is an external view of an endoscope system.
Fig. 2 is an explanatory view illustrating a procedure in a case where an upper alimentary canal is screened.
Fig. 3 is an explanatory view illustrating examination regions defined by guidelines.
Fig. 4 is a block diagram illustrating the functions of the endoscope system.
Fig. 5 is an explanatory view illustrating a measuring scale for measuring the insertion length of an insertion part of the endoscope.
Fig. 6 is an explanatory view illustrating relationships between the insertion lengths of the insertion part of the endoscope, and the examination regions.
Fig. 7 is a table showing relationships between the insertion lengths of the insertion part of the endoscope, and the examination regions (examination sites).
Fig. 8 is a block diagram illustrating the functions of an examination assist mode processing unit.
Fig. 9 is a flowchart illustrating a method of determining an examination image that allows comparison with a reference image.
Fig. 10 is a table illustrating the similarities of reference images and an examination image according to total similarities in which a plurality of feature amounts are combined together.
Fig. 11 is an image view of an examination assist image of which the similarity is displayed.
Fig. 12 is a flowchart illustrating a method of determining whether or not the examination image is an image according to the guidelines depending on its similarity.
Fig. 13 is an image view of an examination assist image in which warning display is performed.
Fig. 14 is an image view of the examination assist image on which operation assist information is displayed.
Fig. 15 is an image view of an examination assist image on which the presence/absence of saving a still image of an examination image is displayed on an examination target map.
Fig. 16 is an image view of an examination assist image in a case where there is a leftover in a partial examination region.
Fig. 17 is an image view of an examination assist image on which the presence/absence of saving still images of examination images is displayed on the examination target map and endoscope operation information is displayed.
Fig. 18 is an image view of an examination assist image on which a reference image is displayed.
Fig. 19 is the image view of an examination assist image on which the reference image, endoscope positional information, and the endoscope operation information are displayed.
Fig. 20 is an image view of an examination assist image on which a reference image is displayed and the presence/absence of saving still images of examination images is displayed on the examination target map.
Fig. 21 is an image view of an examination assist image in which the reference image and the similarity are displayed.
Fig. 22 is an image view of an examination assist image on which the warning display is performed with the display of the reference image.
Fig. 23 is the image view of an examination assist image on which the operation assist information is displayed with the display of the reference image.
Fig. 24 is an image view of an examination assist image on which current and previous examination images are displayed in the same examination region.
Fig. 25 is a block diagram illustrating the functions of an automatic imaging mode processing unit.
Fig. 26 is a table illustrating reference imaging conditions for obtaining 40 reference images defined by the guidelines.
Fig. 27 is a flowchart illustrating a flow in an automatic imaging mode in which a still image of an examination image is automatically acquired.
Fig. 28 is an image view of a monitor displayed in a case where the automatic acquisition of the examination image is performed.
Fig. 29 is a flowchart illustrating a flow in which the still image of the automatically acquired examination image is saved.
Fig. 30 is an image view of the monitor displayed in a case where the still image of the examination image is saved.
Fig. 31 is an image view of the monitor displayed in a case where the still image of the examination image is not saved.
Fig. 32 is a block diagram illustrating the functions of an imaging assist mode processing unit.
Fig. 33 is a flowchart illustrating a flow in an imaging assist mode in which assist of the acquisition timing of the still image of an examination image is performed.
Fig. 34 is an image view of the monitor in which notification of the acquisition timing of the still image is performed.
Fig. 35 is a flowchart illustrating a flow in which the still image is saved in a case where the notification of the acquisition timing of the still image is performed.
Fig. 36 is an explanatory view illustrating the shape of the endoscope in a case where a section directly below the cardia that is an examination region defined by the guidelines is imaged.
Fig. 37 is an explanatory view illustrating the shape of the endoscope in a case where a gastric angle that is an examination region defined by the guidelines is imaged.

### DESCRIPTION OF THE PREFERRED EMBODIMENTS

### First Embodiment

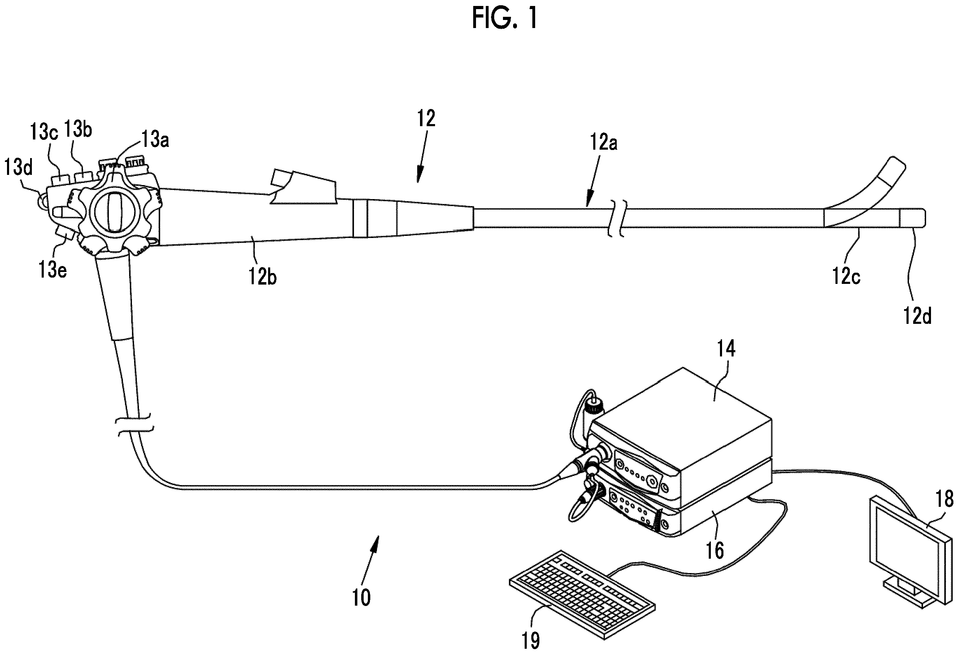

As illustrated in Fig. 1, an endoscope system 10 has an endoscope 12, a light source device 14, a processor device 16, a monitor 18 (display unit), and a console (keyboard) 19. The endoscope 12 is optically connected to the light source device 14, and is electrically connected to the processor device 16. The endoscope 12 has an insertion part 12a to be inserted into a subject, an operating part 12b provided at a proximal end portion of the insertion part 12a, and a bending part 12c and a distal end part 12d provided on a distal end side of the insertion part 12a. By operating an angle knob 13a of the operating part 12b, the bending part 12c makes a bending motion. The distal end part 12d is directed in a desired direction by this bending motion.

Additionally, the operating part 12b is provided with a still image acquisition instruction unit 13b used for operating the acquisition of still images, a mode switching unit 13c used for operating the switching of observation modes, and a zooming operating unit 13d used for operating the change of a zoom magnification factor, in addition to the angle knob 13a.

The endoscope system 10 has an ordinary light mode, a special light mode, and an examination assist mode as the observation modes. In a case where an observation mode is the ordinary light mode, ordinary light such as white light is emitted, and an ordinary light image is displayed on a monitor 18 on the basis of an image signal obtained by imaging an observation object under illumination with this ordinary light. Additionally, in a case where an observation mode is the special light mode, special light with emission spectrum different from that of the ordinary light is emitted, and a special light image is displayed on the monitor 18 on the basis of an image signal obtained by imaging an observation object under illumination with this special light.

Additionally, in a case where an observation mode is the examination assist mode, illumination light for examination is emitted. Also, in order to obtain a reference-equivalent image equivalent to a predetermined reference image for each examination region in guidelines, an examination image is acquired by imaging an observation object within an examination region under illumination with the illumination light for examination. This examination image is displayed on the monitor 18. The examination assist mode is a mode in which an assist is performed so that an image defined by guidelines for medical examination can be reliably captured at screening. In this examination assist mode, it can be confirmed whether or not imaging can be performed according to the guidelines by displaying a similarity with a reference image defined by the guidelines. In addition, the illumination light for examination may be the same as or may be different from the ordinary light or the special light. Additionally, the term "equivalent to" the reference image means that the composition or the like of the reference image is similar.

The processor device 16 is electrically connected to the monitor 18 and the console 19. The monitor 18 outputs and displays an image of the observation object, information accompanying the image, and the like. The console 19 functions as a user interface that receives input operations, such as designation or the like of a region of interest (ROI) and function setting.

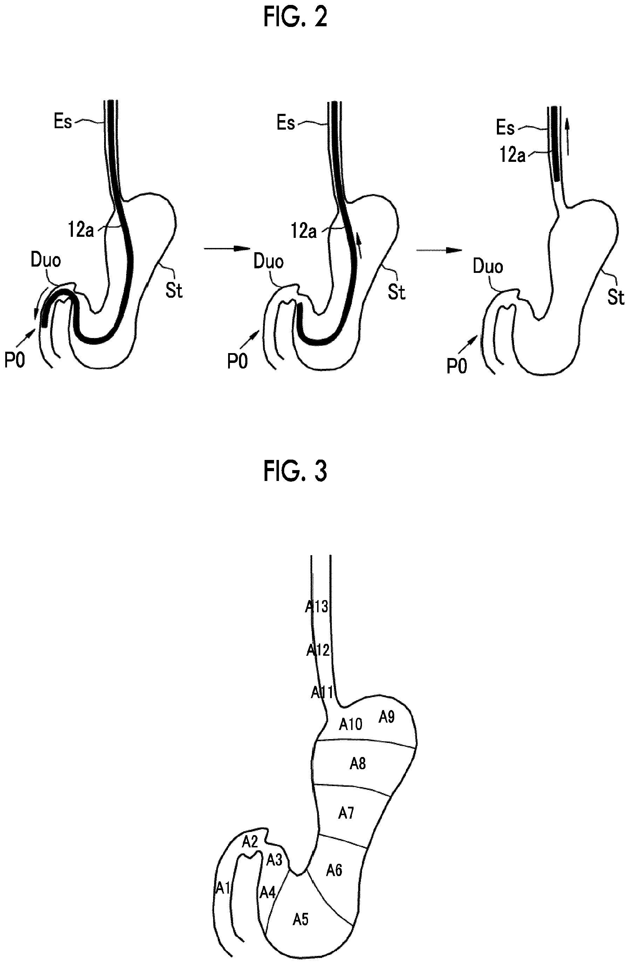

In addition, in a case where an upper alimentary canal, such as the stomach or the esophagus, is screened according to the guidelines, as illustrated in Fig. 2, first, the insertion part 12a of the endoscope 12 is pushed into a lumen, and is inserted into the deepest examination position P0 of a duodenum Duo through an esophagus Es and a stomach St. In a case where the insertion part 12a arrives at the deepest examination position P0, acquisition of still images according to the guidelines is started. First, an operator operates the still image acquisition instruction unit 13b to capture a still image of an observation image in the deepest examination position P0. Thereafter, still images are captured in examination regions defined by the guidelines while the insertion part 12a of the endoscope 12 is gradually pulled out. In a case where imaging of the portions of the duodenum Duo and the stomach St among the examination regions is completed, the insertion part 12a is moved to the esophagus Es, and still images are captured in examination regions of the esophagus Es. In a case where the imaging of these still images is completed, the insertion part 12a is pulled out of a body cavity. In addition, it is preferable to perform screening in the same procedure also regarding a lower alimentary canal, such as the large intestine.

In addition, as the examination regions defined by guidelines, as illustrated in Fig. 3, there are, for example, thirteen examination regions of A1 to A13 regarding the duodenum Duo, the stomach St, and the esophagus Es. A1 and A2 are examination regions of the duodenum Duo, A1 is a duodenal intra posterior part and A2 is a duodenal bulb. A3 to A10 are examination regions of the stomach St, A3 is a pyloric anterior part, A4 is a pyloric antrum, A5 is a gastric angle, A6 is a lower stomach, A7 is a middle stomach, A8 is an upper stomach, A9 is a gastric fundus (a gastric fornicate part), and A10 is a gastric cardia. A11 to A13 are examination regions of the esophagus Es, and, A11 is a lower esophagus, A12 is a middle esophagus, and A13 is an upper esophagus.

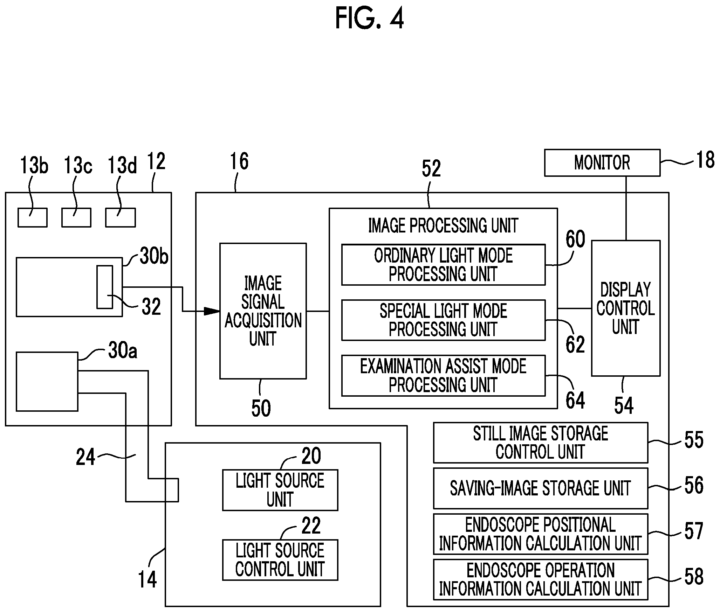

As illustrated in Fig. 4, the light source device 14 includes a light source unit 20 that emits the ordinary light, the special light, or the illumination light for examination, as the illumination light used for the illumination of an observation object, and a light source control unit 22 that controls the light source unit 20. The light source unit 20 is a semiconductor light source, such as a light emitting diode (LED), a xenon lamp, or a halogen lamp. In the light source unit 20, in a case where light of a plurality of colors is emitted, not only LEDs are used for a plurality of colors, but also color separation filters that separate broadband light, such as a xenon lamp, into a plurality of colors are used. The light source control unit 22 controls the quantity of light emission of the illumination light by ON/OFF of LEDs and the adjustment of the driving currents or driving voltages of the LEDs. Additionally, the light source control unit 22 controls the wavelength band of the illumination light, for example, by changing optical filters.

The illumination light emitted from the light source unit 20 enters a light guide 24 inserted into the insertion part 12a via a light path coupling part (not illustrated) formed with a mirror, a lens, or the like. The light guide 24 is built in the endoscope 12 and a universal cord, and propagates the illumination light up to the distal end part 12d of the endoscope 12. The universal cord is a cord that connects the endoscope 12, and the light source device 14 and the processor device 16 together. The distal end part 12d of the endoscope 12 is provided with an illumination optical system 30a and an imaging optical system 30b. The illumination light propagating through the light guide 24 is radiated to the observation object via the illumination optical system 30a.

The imaging optical system 30b has an imaging sensor 32 that various kinds of light, such as reflected light, scattered light, and fluorescence from the observation object, enters. The imaging sensor 32 forms an image of the observation object to output image signals. The output image signal is transmitted to the processor device 16. In addition, the imaging sensor 32 has a monochrome sensor that is not provided with color filters in addition to a color imaging sensor, such as an RGB color imaging sensor or a CMYG color imaging sensor. As the imaging sensor 32, a charge coupled device (CCD) imaging sensor, a complementary metal-oxide semiconductor (CMOS) imaging sensor, or the like is available.

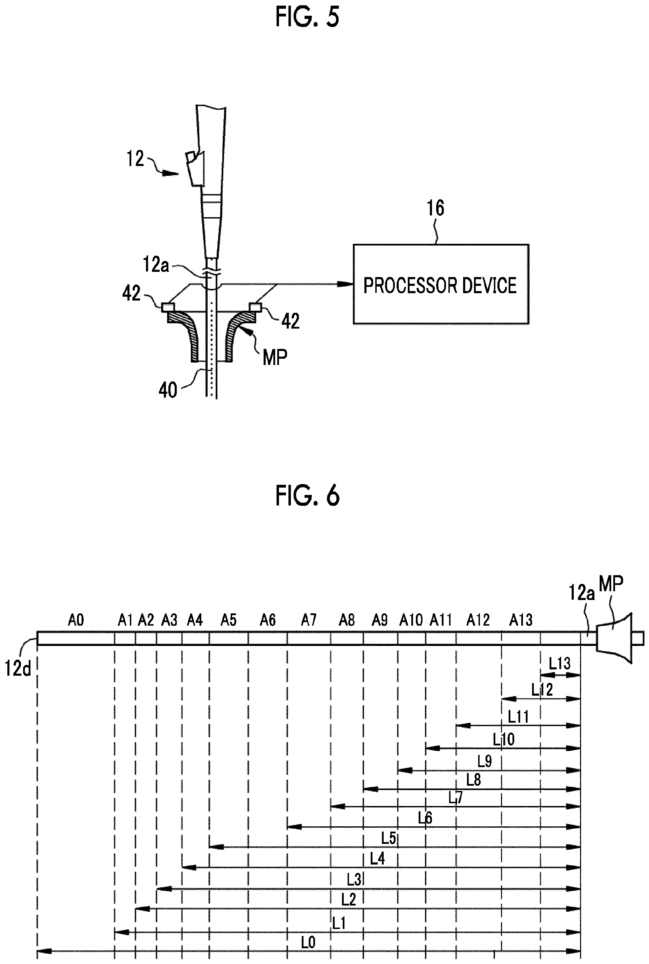

Additionally, as illustrated in Fig. 5, an outer peripheral surface of the insertion part 12a of the endoscope 12 is provided with a measuring scale 40 for measuring the insertion length of the insertion part 12a into the body. The measuring scale 40 is configured of points provided at a predetermined pitch (for example, 1 cm pitch) in a longitudinal direction of the insertion part 12a. The measuring scale 40 is detected by a scale detecting sensor 42 provided in a patient's mouth (in the case of an upper endoscope) and the anus (in the case of a lower endoscope), the scale detecting sensor 42 is wiredly or wirelessly connected to the processor device 16, and detected information in the scale detecting sensor 42 is transmitted to the processor device 16. In addition, in Fig. 5, a mouthpiece MP bitten by a patient's mouth is provided with the scale detecting sensor 42.

As illustrated in Fig. 4, the processor device 16 includes an image signal acquisition unit 50, an image processing unit 52, a display control unit 54, a still image storage control unit 55, a saving-image storage unit 56, an endoscope positional information calculation unit 57, and an endoscope operation information calculation unit 58. The image signal acquisition unit (corresponding to the "image acquisition unit" of the present invention) 50 acquires image signals corresponding to each observation mode from the endoscope 12. The image processing unit 52 includes an ordinary light mode processing unit 60, a special light mode processing unit 62, and an examination assist mode processing unit 64. The ordinary light mode processing unit 60 performs ordinary light mode image processing, such as color conversion processing, color enhancement processing, and structure enhancement processing, on image signals obtained in the ordinary light mode. Accordingly, an ordinary light image with natural color tone is obtained. The ordinary light image is input to the display control unit 54. Additionally, in a case where the still image acquisition instruction unit 13b is operated, a still image of the ordinary light image at that time is stored in the saving-image storage unit 56 by the still image storage control unit 55.

The special light mode processing unit 62 performs different special light mode image processing different from the ordinary light mode image processing on image signals obtained in the special light mode, to obtain a special light image. Although the special light mode image processing is the same as the same processing contents (color conversion processing, color enhancement processing, structure enhancement processing, or the like) as those of an ordinary light mode processing image, these kinds of image processing have different processing conditions. In the special light image, the visibility of a lesion site is higher than that of other sites. The special light image is input to the display control unit 54. Additionally, in a case where the still image acquisition instruction unit 13b is operated, a still image of the special light image at that point of time is stored in the saving-image storage unit 56 by the still image storage control unit 55.

The examination assist mode processing unit 64 performs examination assist mode image processing on image signals obtained in the examination assist mode, to obtain an examination image. The examination image is input to the display control unit 54. In the examination assist mode image processing, in a case where the still image acquisition instruction unit 13b is not operated, the examination image is input to the display control unit 54 as it is. On the other hand, in a case where the still image acquisition instruction unit 13b is operated, an examination assist image for confirming whether or not imaging can be made according to the guidelines is produced. This examination assist image is input to the display control unit 54 together with the examination image. Then, in a case where a user confirms the examination assist image and the still image acquisition instruction unit 13b is further operated, a still image of the examination image at that time is stored in the saving-image storage unit 56 by the still image storage control unit 55. The details of the examination assist mode processing unit 64 will be described below.

The display control unit 54 performs the control of displaying an image input from the image processing unit 52 on the monitor 18. Accordingly, an image corresponding to an observation mode is displayed on the monitor 18. That is, in the ordinary light mode, the ordinary light image is displayed on the monitor 18, and in the special light mode, the special light image is displayed on the monitor 18. Additionally, in the case of the examination assist mode, the examination image is displayed on the monitor 18, and in a case where the still image acquisition instruction unit 13b is operated, the examination assist image is displayed in addition to the examination image.

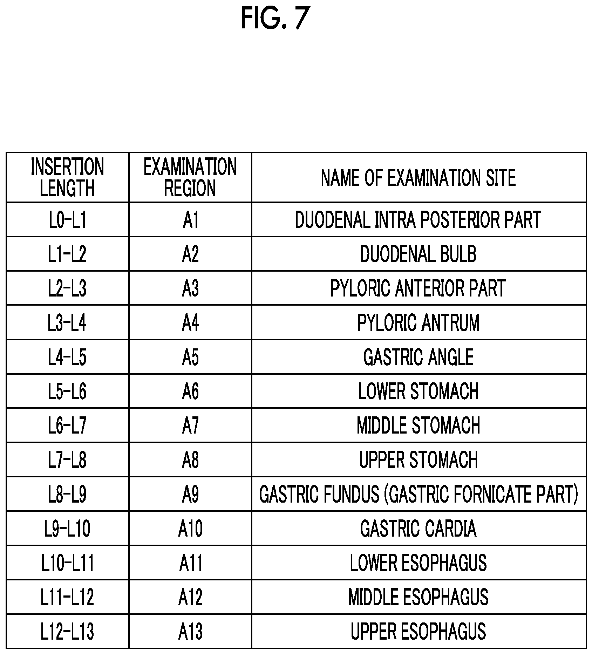

The endoscope positional information calculation unit 57 calculates the insertion length of the insertion part 12a of the endoscope 12 on the basis of the detected information in the scale detecting sensor 42. Additionally, the endoscope positional information calculation unit 57 calculates endoscope positional information showing which of the examination regions A1 to A13 the distal end part 12d of the endoscope 12 is located in, on the basis of the insertion length of the insertion part 12a. In the endoscope positional information calculation unit 57, as illustrated in Figs. 6 and 7, in a case where an insertion length is between L0 and L1, the distal end part 12d is located in the examination region A1, and in a case where an insertion length is between L1 and L2, the distal end part 12d is located in the examination region A2.

Additionally, in a case where an insertion length is between L2 and L3, the distal end part 12d is located in the examination region A3. In a case where an insertion length is between L3 and L4, the distal end part 12d is located in the examination region A4. In a case where an insertion length is between L4 and L5, the distal end part 12d is located in the examination region A5. In a case where an insertion length is between L5 and L6, the distal end part 12d is located in the examination region A6. In a case where an insertion length is between L6 and L7, the distal end part 12d is located in the examination region A7. In a case where an insertion length is between L7 and L8, the distal end part 12d is located in the examination region A8. In a case where an insertion length is between L8 and L9, the distal end part 12d is located in the examination region A9. In a case where an insertion length is between L9 and L10, the distal end part 12d is located in the examination region A10.

Additionally, in a case where an insertion length is between L10 and L11, the distal end part 12d is located in the examination region A11. In a case where an insertion length is between L11 and L12, the distal end part 12d is located in the examination region A12. In a case where an insertion length is between L12 and L13, the distal end part 12d is located in the examination region A13.

In addition, it is preferable to take into consideration positional relationships between the insertion lengths and the examination regions varying depending on the physiques of subjects and calibrate the positional relationships between the insertion lengths and the examination regions depending on physique differences in the endoscope positional information calculation unit 57. For example, an insertion length in the pyloric part like the examination region A3 may be compared with a predetermined pyloric part insertion length for calibration, and a positional relationship between the insertion length and the examination region may be corrected on the basis of a difference between these insertion lengths. Additionally, a distance between the pyloric part like the examination region A3 and a cardiac section of the examination region 10 may be compared with a predetermined distance between the pylorus and the cardia for calibration, and a positional relationship between the insertion length and the examination region may be corrected on the basis of a difference between these distances.

The endoscope operation information calculation unit 58 calculates endoscope operation information showing information on the operation of the endoscope 12. The bending angle or the rotational amount of the distal end part 12d of the endoscope 12, a distance (observation distance) from an observation object, insertion length, the shape of the endoscope, and the like are included in the endoscope operation information. The bending angle or the rotational amount is calculated on the basis of the operation information of the operating parts 12b, such as the operation amount of the angle knob 13a. Additionally, the observation distance is calculated on the basis of an average value of pixel values of an examination image, or the like. The insertion length is calculated by the endoscope positional information calculation unit 57 as described above. It is preferable that the shape of the endoscope is calculated using a magnetic sensor (not illustrated) provided at distal end part 12d of the endoscope. A plurality of the magnetic sensors may be provided at the insertion part 12a at regular intervals from the distal end part 12d, in addition to the distal end part 12d.

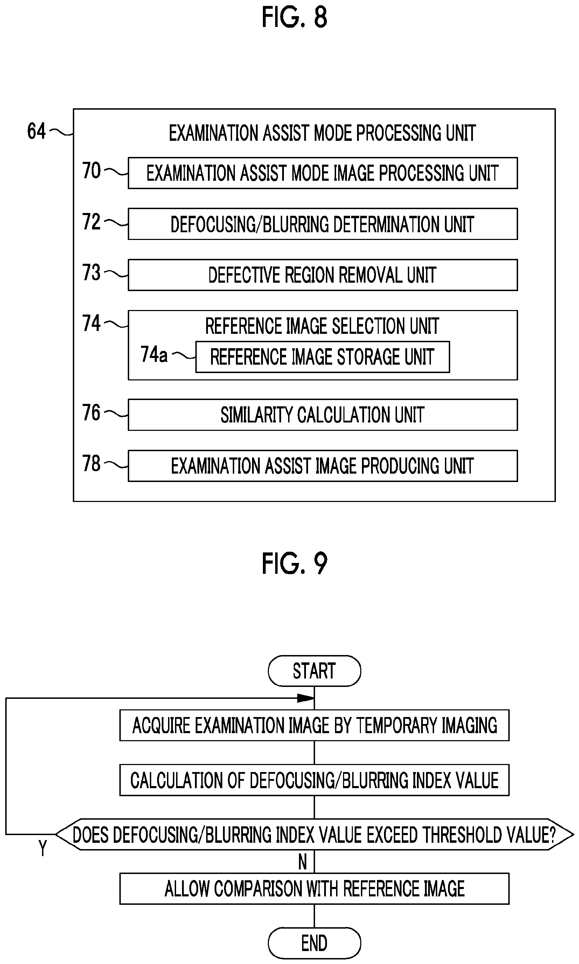

As illustrated in Fig. 8, the examination assist mode processing unit 64 has a defocusing/blurring determination unit 72, a defective region removal unit 73, a reference image selection unit 74, a similarity calculation unit 76, and an examination assist image producing unit 78, as processing units that operate in a case where the still image acquisition instruction unit 13b is operated, in addition to an examination assist mode image processing unit 70 that performs the examination assist mode image processing. In addition, in the examination assist mode, it is confirmed whether or not a still image of an examination image is an image according to the guidelines, and then the still image is stored in the saving-image storage unit 56.

For that reason, imaging the still image of the examination image is performed in two steps including temporary imaging and main imaging. The default is set to the state of the temporary imaging, and the temporary imaging is performed in a case where the still image acquisition instruction unit 13b is operated. After this temporary imaging, in a case where the still image acquisition instruction unit 13b is further operated, the main imaging is performed and a still image of an acquired examination image is stored in the saving-image storage unit 56. After being stored in the saving-image storage unit 56, return to the state of the temporary imaging is made.

As illustrated in Fig. 9, the defocusing/blurring determination unit 72 determines whether or not defocusing or blurring occurs in a still image of an examination image subjected to the temporary imaging. In the defocusing/blurring determination unit 72, in order to determine defocusing or blurring, a defocusing/blurring index value showing defocusing or blurring of the still image of the examination image subject to the temporary imaging is calculated. As a method of calculating the defocusing/blurring index value, for example, there is a method of calculating the defocusing/blurring index value on the basis of the spatial frequency of an examination image because the defocusing or blurring is related to the spatial frequency of the image.

In a case where the calculated defocusing/blurring index value exceeds a threshold value, the defocusing/blurring determination unit 72 calculates that defocusing/blurring occurs in the examination image. Since it is difficult to accurately compare the examination image in which the defocusing/blurring occurs in this way with a reference image, the guidance of prompting the user to re-capture an examination image is displayed on the monitor 18. On the other hand, in a case where the calculated defocusing/blurring index value is equal to or lower than the threshold value, the defocusing/blurring determination unit 72 determines that the defocusing/blurring does not influence the comparison with the reference image, or the like, and allows the comparison with the reference image. In this case, the examination image is transmitted to the defective region removal unit 73.

The defective region removal unit 73 removes a defective region, such as dirt or halation on an observation object, from the examination image. For example, a high pixel region of which pixel values are equal to or higher than a certain value in an examination image is recognized as a halation region, and the pixel values of this region are replaced with the pixel values of an adjacent region. The examination image after the removal of the defective region is transmitted to the reference image selection unit 74.

The reference image selection unit 74 includes a reference image storage unit 74a that stores a plurality of predetermined reference images for each of the examination regions A1 to A13. The reference image selection unit 74 performs comparison processing between the reference images stored in the reference image storage unit 74a and an examination image subjected to defocusing/blurring determination or defective region removal. The comparison processing is performed by pattern matching or the like. On the basis of this comparison processing, a reference image corresponding to the examination image is selected from the reference image storage unit 74a. The selected reference image and the examination image are transmitted to the similarity calculation unit 76.

Additionally, in the reference image storage unit 74a, the reference images are stored in RAW data format such that the statuses of the reference images can be changed in conformity with the examination assist mode image processing that is being used in the examination assist mode processing unit 64. Hence, in the reference image selection unit 74, before comparison processing with an examination image is performed, the examination assist mode image processing (corresponding to "specific image processing" of the present invention) is performed on the reference images stored in the reference image storage unit 74a, and then, comparison processing between the reference images subjected to the examination assist mode image processing and the examination image is performed. In addition, in the reference image selection unit 74, comparison processing between an image before the examination assist mode image processing is performed, and the reference images in RAW data format may be performed. Additionally, the RAW data means image data in a state where various kinds of image processing (in the present embodiment, the ordinary light mode image processing, the special light mode image processing, and the examination assist mode image processing), such as tone enhancement processing, are not performed.

The similarity calculation unit 76 calculates a similarity between a reference image and an examination image on the basis of image data of the reference image and image data of the examination image. The calculated similarity with the reference image is transmitted to the examination assist image producing unit 78. As a method of calculating the similarity, for example, noise included in the image data of the reference image is compared with noise included in the image data of the examination image, and it is assumed that the similarity is higher as a difference between the amounts of these kinds of noise is smaller. Additionally, the structure of creases or the like included in the image data of the reference image is compared with the structure included in the image data of the examination image or a landmark, such as the cardia or the pylorus, and it is estimated that the similarity is higher in a case where there are more similar structures. Additionally, in a case where the insertion part 12a of the endoscope 12 is projected on the reference image, it is preferable to determine the similarity depending on the presence/absence of the insertion part 12a. In this case, the similarity is made high in a case where the insertion part 12a is included in the examination image. Moreover, in addition to comparing the image data of the reference image with the image data of the examination image, the endoscope operation information may also be added to calculate the similarity. In this way, the similarity can be accurately calculated by adding the endoscope operation information.

Additionally, as a method of calculating the similarity, a method using the feature amount of a color or the like of an image is conceivable. However, since it is difficult to accurately calculate only one feature amount and the similarity, the similarity can be accurately calculated using a total similarity in which a plurality of feature amounts are combined together. For example, in a case where a feature amount A of a color, a feature amount B of a structure, and a feature amount C of a landmark are used as the feature amounts, the total similarity is calculated by weighing and adding these feature amounts A to C.

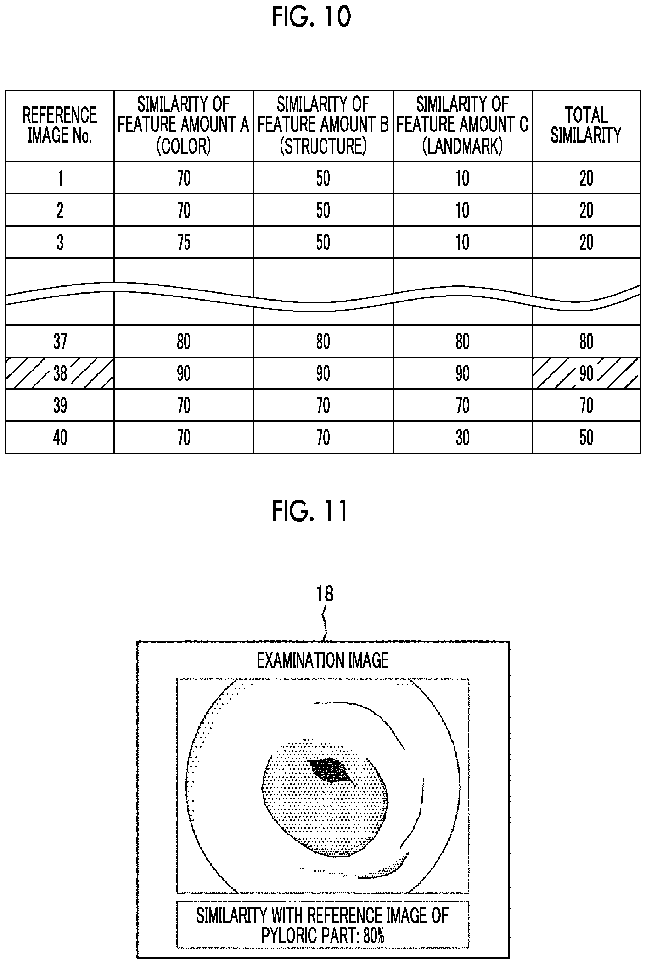

In addition, the above total similarity may be used for selection of a reference image, in the reference image selection unit 74. For example, as illustrated in Fig. 10, in a case where there are 1 to 40 reference images, the similarities (in Fig. 10, the similarities are expressed in % (percentage)) of the feature amounts A to C with an examination image are calculated regarding the 40 reference images, and total similarities are calculated on the basis of the calculated similarities of the feature amounts A to C. Then, a reference image of which the total similarity is the highest is selected. In this case, since the reference image of which the total similarity is the highest is reference image No. 38, this reference image No. 38 is selected.

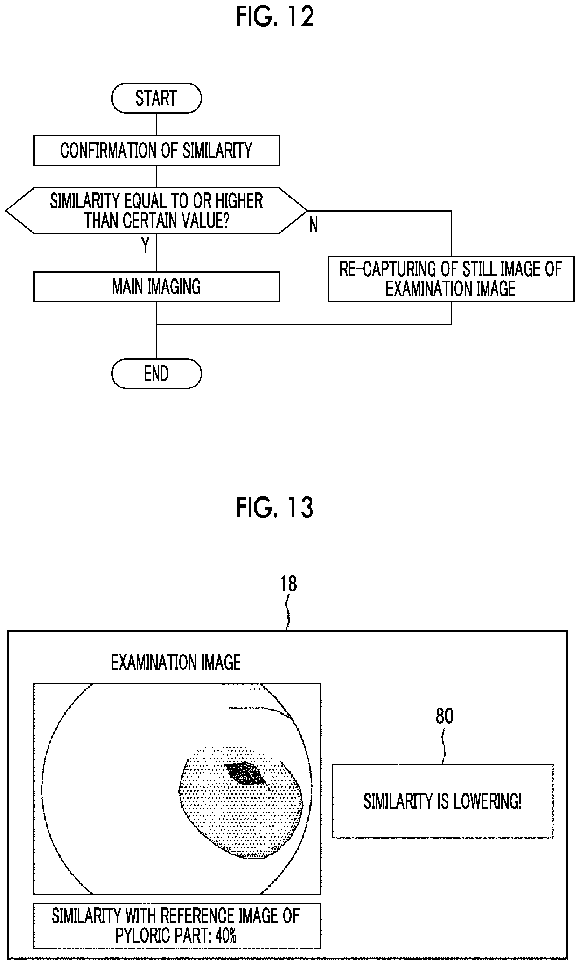

The examination assist image producing unit 78 produces an examination assist image on the basis of the similarity between the examination image and the reference image. The produced examination assist image is transmitted to the display control unit 54, and is displayed on the monitor 18. As illustrated in Fig. 11, in an examination assist image, the similarity with a reference image (the similarity with a reference image of a pyloric part is "80%") is displayed together with an examination image. As illustrated in Fig. 12, the user confirms the similarity between the examination image and the reference image, and in a case where it is confirmed that the similarity satisfies a specific condition (for example, equal to or higher than a certain value), the examination image is determined to be an image (reference-equivalent image) according to the guidelines, and performs the main imaging of operating the still image acquisition instruction unit 13b again. Accordingly, the examination image is stored in the saving-image storage unit 56. In addition, in a case where the similarity of the examination image with the reference image does not satisfy the specific condition, such as equal to or higher than the certain value, as illustrated in Fig. 13, a warning display 80 may be performed on the monitor 18 by the display control unit 54.

On the other hand, in a case where the user determines that the examination image is not the image (reference-equivalent image) according to the guidelines, such as in a case where the similarity does not satisfy the specific condition, the storage of the examination image in the saving-image storage unit 56 is suspended by not operating the still image acquisition instruction unit 13b for a certain time or more or by operating an examination image rejection button (not illustrated) provided in the endoscope 12 or the console 19. In this case, the display of the examination assist image is stopped and a still image of the examination image is captured again.

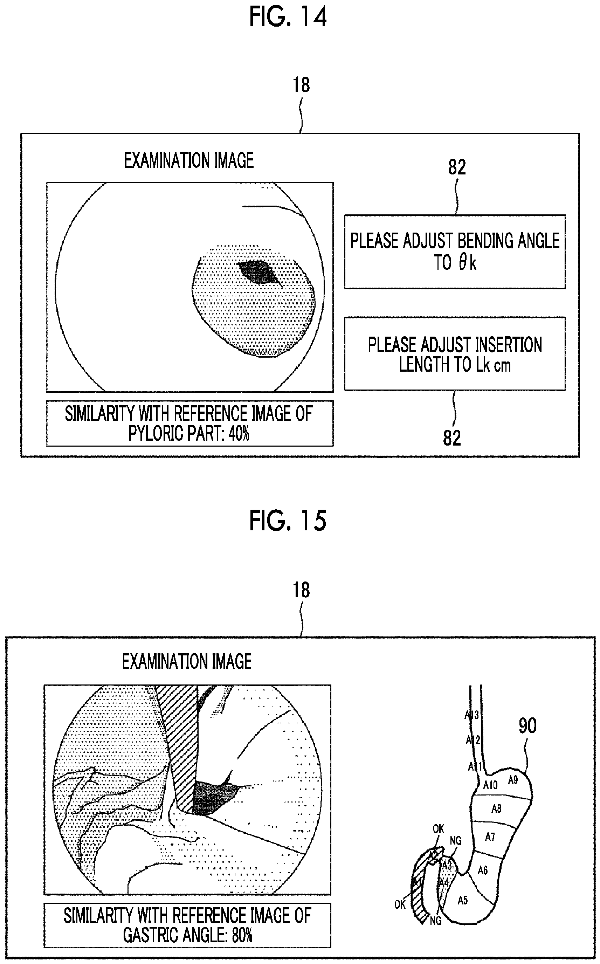

In a case where the still image of the examination image is captured again, it is preferable that the operation assist information for assisting the operation of the endoscope 12 is displayed on the monitor 18 by the display control unit 54 so that a user can acquire an examination image according to the guidelines (an examination image of which the similarity satisfies the specific condition). Information on how much the bending angle or the insertion length should be adjusted or information on how much the air-supply pressure of supply gas to be blown against an observation object should be set, in order to obtain the examination image according to the guidelines, is included as the operation assist information. For example, in the case of Fig. 14, operation assist information 82 on the bending angle and the insertion length is displayed. In a case where the similarity of the examination image with the reference image does not satisfy the specific condition, the operation assist information is automatically displayed on the monitor 18.

In addition, in addition to the display of the similarity between the examination image and the reference image, a display in which it is possible to know whether or not the acquisition of the examination image has succeeded or failed in the respective examination regions may be performed on the examination assist image. As illustrated in Fig. 15, in an examination assist image, in addition to the similarity between an examination image and a reference image, a display regarding the presence/absence of saving examination images into the saving-image storage unit 56 may be performed by the display control unit 54 on an examination target map 90 in which the examination regions A1 to A13 are mapped. In this examination assist image, a display indicating that examination images of the examination regions A1 and A2 have been saved (the display of "OK" display in Fig. 15) is performed, and a display indicating that still images of examination images of the examination regions A3 and A4 have been acquired but the saving of the still images into the saving-image storage unit 56 is not formed (the display of "NG" in Fig. 15) is performed. Here, it is preferable that the regions of OK and the regions of NG are expressed in different colors, respectively, (for example, the regions of OK are displayed in "blue" and the regions of NG are displayed in "yellow"). In addition, information on the examination regions is acquired from the endoscope positional information.

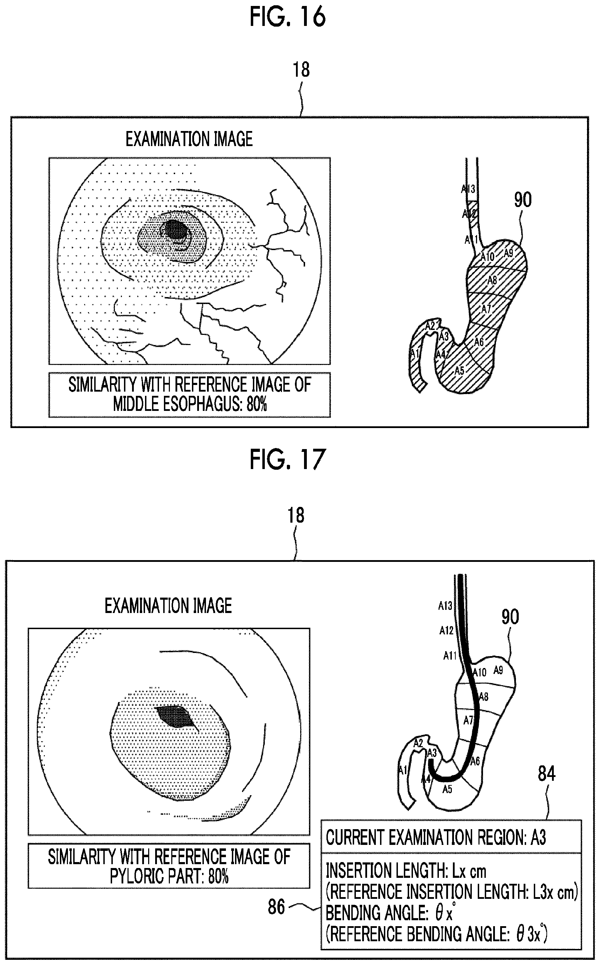

In addition, in the examination assist image, in a case where the presence/absence of saving the still images of the examination images is displayed, it is possible to discover failures of capturing the examination images. As illustrated in Fig. 16, it can be seen that, in a case where examination images of the examination regions A1 to A10 and the examination region A12 are displayed as saved and the examination region A11 between A10 and A12 is not displayed as saved, the examination region A11 (lower esophagus) could not be imaged. In such a case, the insertion part 12a of the endoscope 12 is pushed again so as to image the examination region A11 (lower esophagus).

In addition, as illustrated in Fig. 17, in addition to the display of the similarity between an examination image and a reference image, information on endoscope operation information 84 or endoscope positional information 86 may be displayed on an examination assist image. In this examination assist image, as the endoscope operation information, the insertion length and the bending angle are displayed, and a reference insertion length and a reference bending angle in a case where the reference image is obtained are also displayed together. Additionally, which examination region the distal end part 12d of the endoscope is located in as the endoscope positional information is displayed (for example, the examination region A3). Moreover, in the examination assist image, a display as to what kind of shape the insertion part 12a is inserted in the stomach that is an object to be examined is performed using the shape of the insertion part 12a of the endoscope 12 in the endoscope operation information.

### Second Embodiment

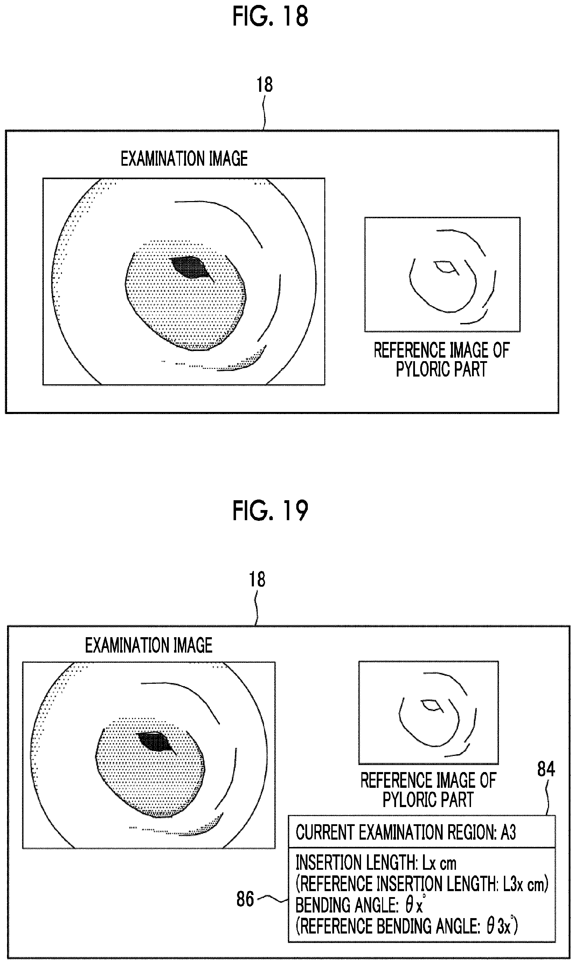

In the first embodiment, in the examination assist image, the similarity with the reference image is displayed with the examination image. However, in the second embodiment, as illustrated in Fig. 18, instead of the display of the similarity with the reference image, the reference image (for example, the reference image of the pyloric part) may be displayed. In this case, the user operates the endoscope 12 such that the examination image have the same composition as the reference image, and operates the still image acquisition instruction unit 13b to acquire a still image of the examination image in a case where the examination image and the reference image has the same structure. Moreover, as illustrated in Fig. 19, in addition to the examination image and the reference image, the information on the endoscope operation information 84 and the endoscope positional information 86 may also be displayed together on the examination assist image. In this case, the user operates the endoscope 12 while viewing the endoscope operation information, and matches the structure of the examination image with the structure of the reference image.

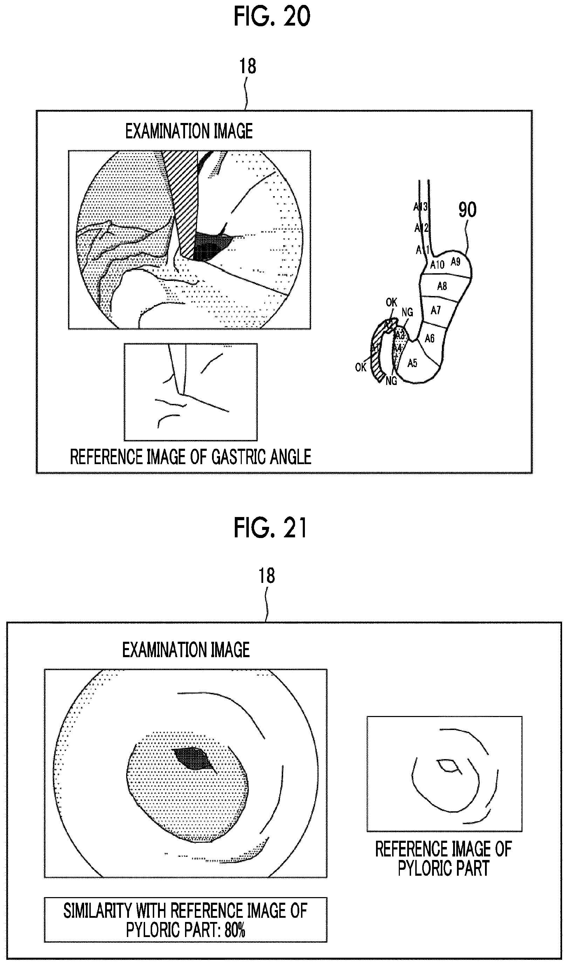

Additionally, as illustrated in Fig. 20, in an examination assist image, in addition to the examination image and the reference image, the examination target map 90 may be displayed, and a display regarding the presence/absence of saving examination images into the saving-image storage unit 56 may be performed on the examination target map 90. In the display regarding the presence/absence of the saving, in the first embodiment, OK is displayed regarding examination regions where the saving of the examination images into the saving-image storage unit 56 is completed, and NG is displayed regarding examination regions where still images of the examination images are acquired but the saving of the examination image into the saving-image storage unit 56 is not completed.

Additionally, as illustrated in Fig. 21, in an examination assist image, in addition to the examination image and the reference image (for example, the reference image of the pyloric part), the similarity with the reference image (the similarity with the reference image of the pyloric part is "80%") may be calculated and displayed. In this case, an image (reference-equivalent image) according to the guidelines can be more reliably captured by using not only the reference image but the similarity. In a case where the similarity is calculated in this way, in the second embodiment, various kinds of user assist in a case where the similarity does not satisfy the specific conditions become possible.

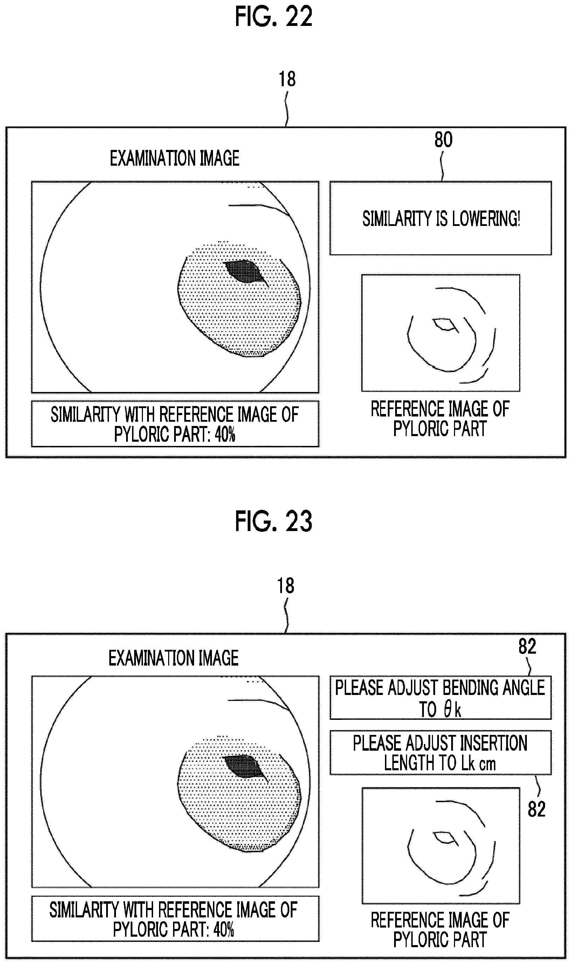

For example, as illustrated in Fig. 22, in an examination assist image, in addition to the examination image, the reference image, and the similarity, the warning display 80 indicating that the similarity does not satisfy the specific condition may be performed. Additionally, as illustrated in Fig. 23, in an examination assist image, the operation assist information 82 required for the imaging of the examination image according to the guidelines may be displayed. In this case, by displaying the warning display 80 and the operation assist information 82 in addition to the reference image and the similarity, an image (reference-equivalent image) according to the guidelines can be more reliably captured by using not only the reference image but the similarity. Moreover, assist in the operation of the endoscope may be performed by adding a function to turn on a light emitting member, such as a light emitting diode (LED) built in the angle knob 13a of the operating part 12b so that a rotational direction or the like can be known.

In addition, in the above embodiment, in a single endoscopic examination, a similarity with a reference image or the reference image may be displayed such that imaging can be performed according to the guidelines. However, it is preferable to display a plurality of examination images with different acquisition timings in the same examination region on the monitor 18 so that effective diagnosis can be performed also in the subsequent follow-up observation. In this case, in order to allow a lesion site to be found in an initial examination and then allow the follow-up observation to be performed, in a case where the examination images are stored in the saving-image storage unit 56 in the initial examination, it is preferable to store the endoscope positional information or the endoscope operation information in an associated manner.

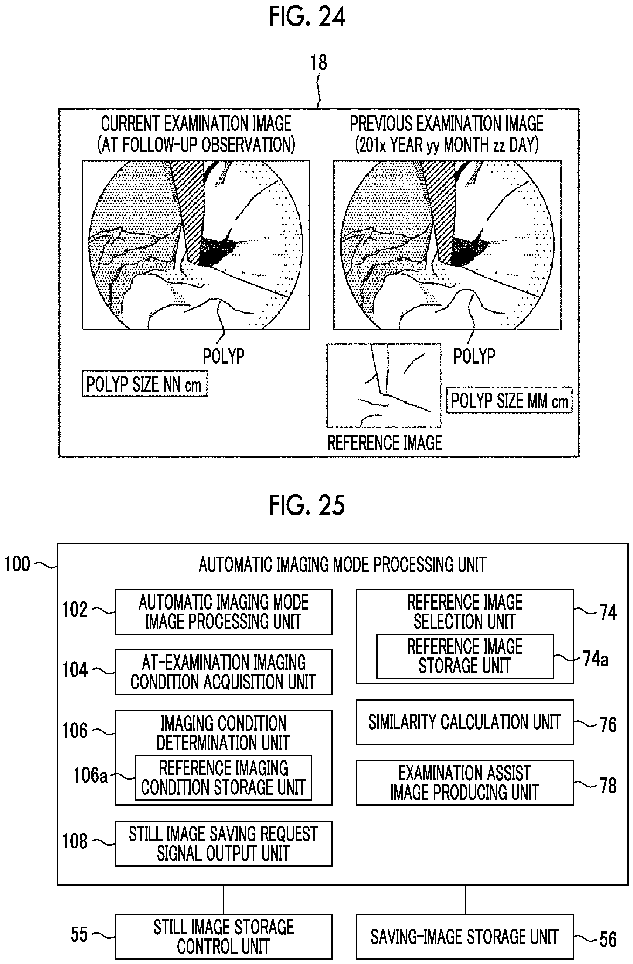

As illustrated in Fig. 24, at the time of the follow-up observation, the endoscope positional information or the endoscope operation information on a position where a lesion site is discovered is set in advance, and in a case where distal end part 12d of the endoscope arrives at the position where the lesion site is discovered, a previous examination image is displayed with an examination image. Additionally, in addition to the previous examination image, the size of the lesion site (polyp) at the current follow-up observation and the size of the lesion site (polyp) at the previous follow-up observation are displayed. Moreover, a reference image may be displayed.

### Third Embodiment

In the first and the second embodiment, in a case where the still image of the examination image is acquired in the temporary imaging by the user, the similarity with the reference image or the reference image is displayed, the similarity or the reference image is confirmed, and then the main imaging is performed to store the still image of the examination image in the saving-image storage unit. However, instead of this, in a third embodiment, in a case where an at-examination imaging condition obtained at an acquisition timing of an examination image satisfies a reference imaging condition for obtaining a reference image during the display of the examination image, the temporary imaging is automatically performed to acquire a still image of the examination image.

In the endoscope system 10 of a third embodiment, an automatic imaging mode in which a still image of an examination image is automatically acquired in a case where the at-examination imaging condition satisfies a reference imaging condition is provided instead of the examination assist mode. For that reason, as illustrated in Fig. 25, in the processor device 16 of the endoscope system 10, an automatic imaging mode processing unit 100 is provided instead of the examination assist mode processing unit 64 of the first and second embodiments. In addition, switching to the automatic imaging mode is performed by the mode switching unit 13c.

The automatic imaging mode processing unit 100 includes an automatic imaging mode image processing unit 102, an at-examination imaging condition acquisition unit 104, an imaging condition determination unit 106, and a still image saving request signal output unit 108, in addition to the reference image selection unit 74, the similarity calculation unit 76, and the examination assist image producing unit 78 that are illustrated in the first and second embodiments. In addition, in the third embodiment, the defocusing/blurring determination unit 72 and the defective region removal unit 73 are not provided because the comparison between an examination image and reference images is not performed. However, in a case where the comparison between an examination image and reference images is performed in addition to the comparison between an at-examination imaging condition and reference imaging conditions, it is preferable to provide the defocusing/blurring determination unit 72 and the defective region removal unit 73 together.

The automatic imaging mode image processing unit 102 performs automatic imaging mode image processing on an image signal obtained in the automatic imaging mode. The automatic imaging mode image processing is the same as the examination assist mode image processing of the first and second embodiments, and an examination image is obtained by performing this automatic imaging mode image processing. The examination image is input to the display control unit 54, and is displayed as a moving image on the monitor 18.

The at-examination imaging condition acquisition unit 104 acquires an imaging condition in a case where the examination image is acquired, as an at-examination imaging condition. At least any of endoscope positional information or endoscope operation information in a case where the examination image is acquired is included in the at-examination imaging condition. Hence, whenever an examination image equivalent to a predetermined frame is acquired, the at-examination imaging condition acquisition unit 104 makes an access to the endoscope positional information calculation unit 57 and the endoscope operation information calculation unit 58, and acquires the endoscope positional information or the endoscope operation information.

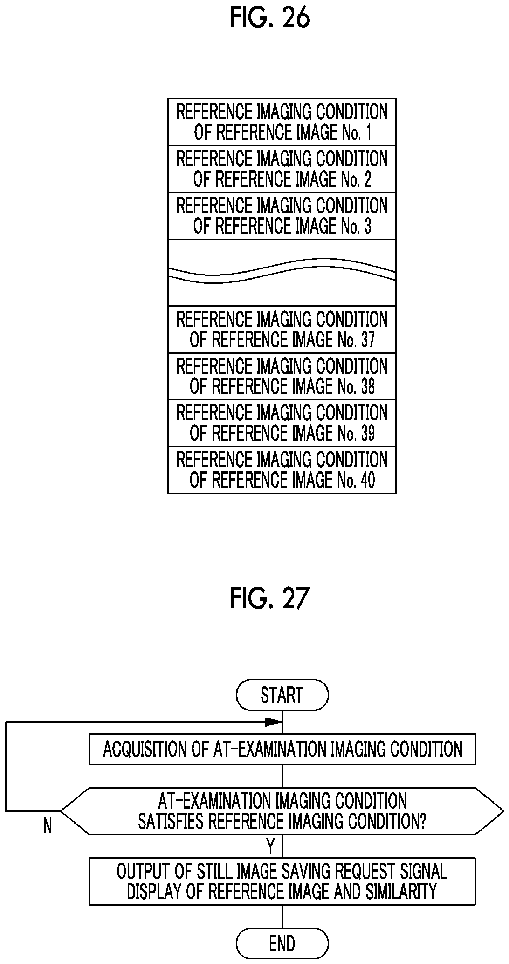

The imaging condition determination unit 106 determines whether or not the at-examination imaging condition satisfies a reference imaging condition for obtaining a reference image. The imaging condition determination unit 106 includes a reference imaging condition storage unit 106a that stores reference imaging conditions for obtaining the reference images defined by the guidelines. For example, in a case where there are 1 to 40 reference images, as illustrated in Fig. 26, a reference imaging condition of reference image No. 1 is stored as a reference imaging condition for obtaining reference image No. 1, and also regarding other reference image No. 2 to reference image No. 40, a reference imaging condition of reference image No. 2 to a reference imaging condition of reference image No. 40 are stored as reference imaging conditions for obtaining these reference images.

At least any of endoscope positional information or endoscope operation information for obtaining the reference images is included in the reference imaging conditions. Additionally, the at-examination imaging condition satisfying a reference imaging condition means the followings. In a case where an imaging condition is insertion length and in a case where an insertion length Lx in a case where an examination image is acquired is included in a certain range Ly1 to Ly2 (Ly2 > Ly1) including an insertion length Ly for obtaining a predetermined reference image, it is said that the at-examination imaging condition satisfies a reference imaging condition.

In case where the imaging condition determination unit 106 determines that the at-examination imaging condition satisfies any of the plurality of reference imaging conditions stored in the reference imaging condition storage unit 106a, as illustrated in Fig. 27, the still image saving request signal output unit 108 outputs a still image saving request signal, which requests the saving of a still image of an examination image into the saving-image storage unit 56, to the display control unit 54 or the still image storage control unit 55. In addition, in a case where the imaging condition determination unit 106 determines that the at-examination imaging condition does not satisfy any of the reference imaging conditions, the user operates the endoscope 12 such that the endoscope 12 is located in an examination site defined by the guidelines, and performs re-acquisition of the at-examination imaging condition.

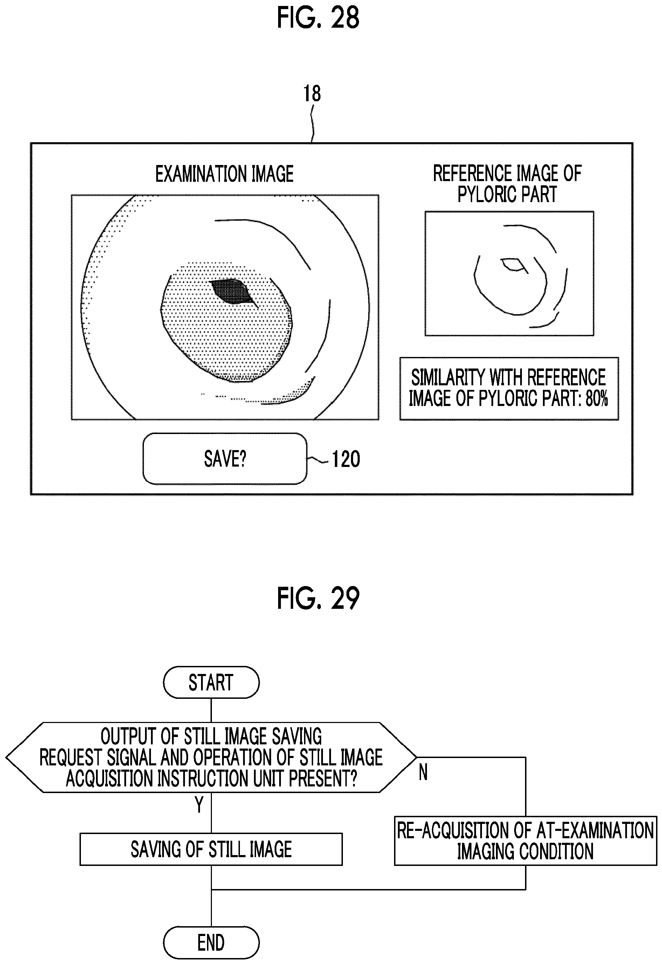

The display control unit 54 receives the still image saving request signal, and as illustrated in Fig. 28, causes a confirmation message 120 for confirming whether or not an examination image is saved as a still image to be displayed on the monitor 18, together with the examination image. Additionally, in a case where the still image saving request signal is output, the reference image selection unit 74 selects a reference image corresponding to a reference imaging condition that satisfies the at-examination imaging condition from the reference image storage unit 74a. This selected reference image is displayed on the monitor 18 by the display control unit 54 ("the reference image of the pyloric part" is displayed in Fig. 28). In addition, in the reference image storage unit 74a of the third embodiment, the reference images are stored in association with the reference imaging conditions so that a reference image can be selected from the reference imaging conditions.

Additionally, the similarity calculation unit 76 calculates a similarity between the reference image selected by the reference image selection unit 74 at the time of still image saving request signal output, and the examination image. The calculated similarity is displayed on the monitor 18 by the display control unit 54 ("similarity (80%) with the reference image of the pyloric part" is displayed in Fig. 28).

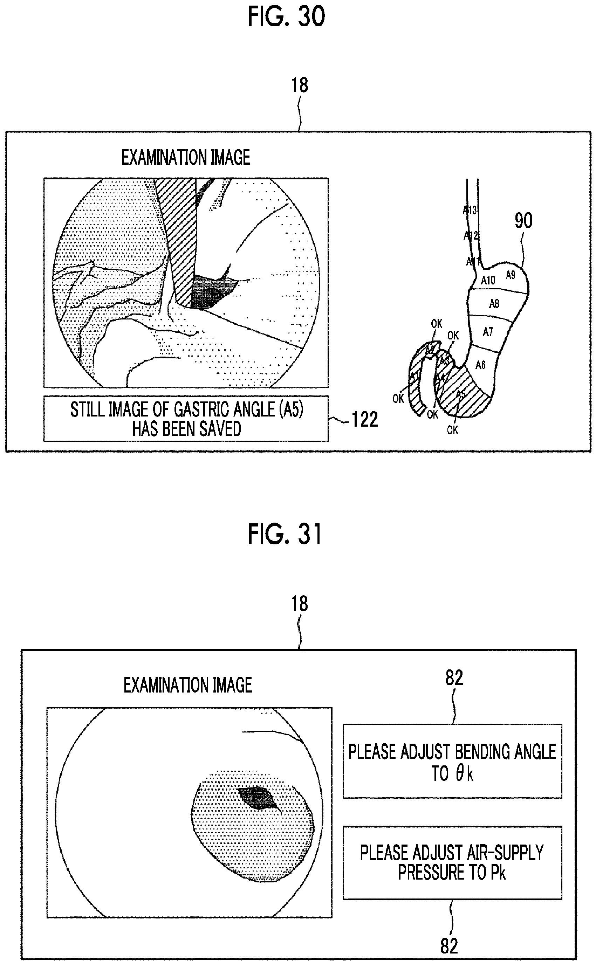

As illustrated in Fig. 29, the still image storage control unit 55 performs the saving of the still image of the examination image into the saving-image storage unit 56 in a case where the still image saving request signal is received, and the user determines that the still image of the examination image displayed on the monitor 18 is an image (reference-equivalent image) according to the guidelines and performs the operation of the still image acquisition instruction unit 13b. In a case where the still image of the examination image is saved, the display control unit 54 causes a saving completion message 122, indicating that the still image is saved, to be displayed on the monitor 18, as illustrated in Fig. 30. Additionally, the display control unit 54 causes an examination region, where the still image is saved to be displayed, on the examination target map 90. In the examination target map 90, OK is displayed in the examination region, where the saving of the still image is saved, among the examination regions A1 to A13.

On the other hand, the still image storage control unit 55 does not perform the saving of the examination image into the saving-image storage unit 56 in a case where the still image saving request signal is received, but the user determines that the still image of the examination image displayed on the monitor 18 is not the image (reference-equivalent image) according to the guidelines and does not perform the operation of the still image acquisition instruction unit 13b within a given time from the reception of the still image saving request signal. Additionally, the saving of the examination image into the saving-image storage unit 56 is not performed even in a case where the still image saving request signal is received, but the examination image rejection button (not illustrated) provided on the endoscope 12 or the console 19 is operated. In addition, in a case where the still image of the automatically acquired examination image is not saved, switching to a manual imaging mode in which the still image of the examination image is manually acquired is automatically performed. In this case, it is preferable to display a message indicating the switching to the manual imaging mode on the monitor 18.

As described above, in a case where the saving of the examination image into the saving-image storage unit 56 is not performed, the display of the confirmation message 120 for confirming whether or not the still image is saved is also stopped. Instead of the confirmation message 120, as illustrated in Fig. 31, the operation assist information 82 required for the imaging of an image (reference-equivalent image) according to the guidelines is displayed on the monitor 18. Then, the user operates the endoscope 12 to perform re-acquisition of the at-examination imaging condition while confirming the operation assist information 82 on the monitor 18.

### Fourth Embodiment

In the third embodiment, in a case where the at-examination imaging condition satisfies the reference imaging condition, a still image of the examination image is automatically acquired. However, in a fourth embodiment, in a case where an at-examination imaging condition satisfies a reference imaging condition, assist in the acquisition (imaging) of a still image of an examination image by a user is performed by notifying the user of an acquisition timing of the still image of the examination image, that is, a shutter timing.

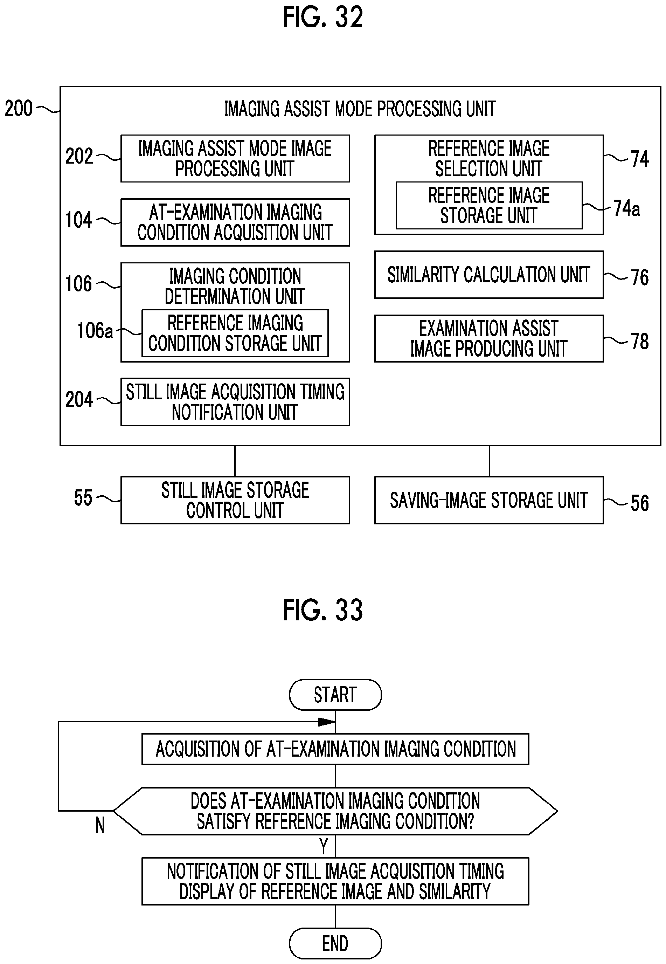

In the endoscope system 10 of the fourth embodiment, an imaging assist mode in which notification of an acquisition timing of a still image of an examination image is performed in a case where an at-examination imaging condition satisfies a reference imaging condition is provided instead of the examination assist mode of the first and second embodiments. For that reason, as illustrated in Fig. 32, in the processor device 16 of the endoscope system 10, an imaging assist mode processing unit 200 is provided instead of the examination assist mode processing unit 64 of the first and second embodiments. In addition, switching to the imaging assist mode is performed by the mode switching unit 13c. Additionally, in the fourth embodiment, in addition to the imaging assist mode, the automatic imaging mode of the third embodiment may be provided such that mode switching is performed between the imaging assist mode and the automatic imaging mode.

The imaging assist mode processing unit 200 includes an imaging assist mode image processing unit 202 and a still image acquisition timing notification unit 204, in addition to the reference image selection unit 74, the similarity calculation unit 76, and the examination assist image producing unit 78 that are illustrated in the first and second embodiments and the at-examination imaging condition acquisition unit 104 and the imaging condition determination unit 106 that are illustrated in the third embodiment. In addition, in the fourth embodiment, the defocusing/blurring determination unit 72 and the defective region removal unit 73 are not provided because the comparison between an examination image and a reference image is not performed. However, in a case where the comparison between an examination image and a reference image is performed in addition the comparison between the at-examination imaging condition and the reference imaging condition, it is preferable to provide the processing by the defocusing/blurring determination unit 72 or the defective region removal unit 73 together.

The imaging assist mode image processing unit 202 performs imaging assist mode image processing on an image signal obtained in the imaging assist mode. The imaging assist mode image processing is the same as the examination assist mode image processing of the first and second embodiments, and an examination image is obtained by performing this imaging assist mode image processing. The examination image is input to the display control unit 54, and is displayed as a moving image on the monitor 18.

Also in the fourth embodiment, similar to the third embodiment, the at-examination imaging condition acquisition unit 104 acquires an imaging condition in a case where the examination image is acquired, as an at-examination imaging condition. Then, the imaging condition determination unit 106 determines whether or not the acquired at-examination imaging condition satisfies a reference imaging condition. In addition, at least any of endoscope positional information or endoscope operation information in a case where the examination image is acquired is included in the at-examination imaging condition. Hence, whenever an examination image equivalent to a predetermined frame is acquired, an access to the endoscope positional information calculation unit 57 and the endoscope operation information calculation unit 58 is made, and the endoscope positional information or the endoscope operation information is acquired.

In a case where the imaging condition determination unit 106 determines that the at-examination imaging condition satisfies a reference imaging condition, the still image acquisition timing notification unit 204 performs the notification of reaching the acquisition timing of the still image of the examination image, as illustrated in Fig. 33. In addition, in a case where the imaging condition determination unit 106 determines that the at-examination imaging condition does not satisfy any of the reference imaging conditions, the user operates the endoscope 12 such that the endoscope 12 is located in an examination site defined by the guidelines, and performs re-acquisition of the at-examination imaging condition.

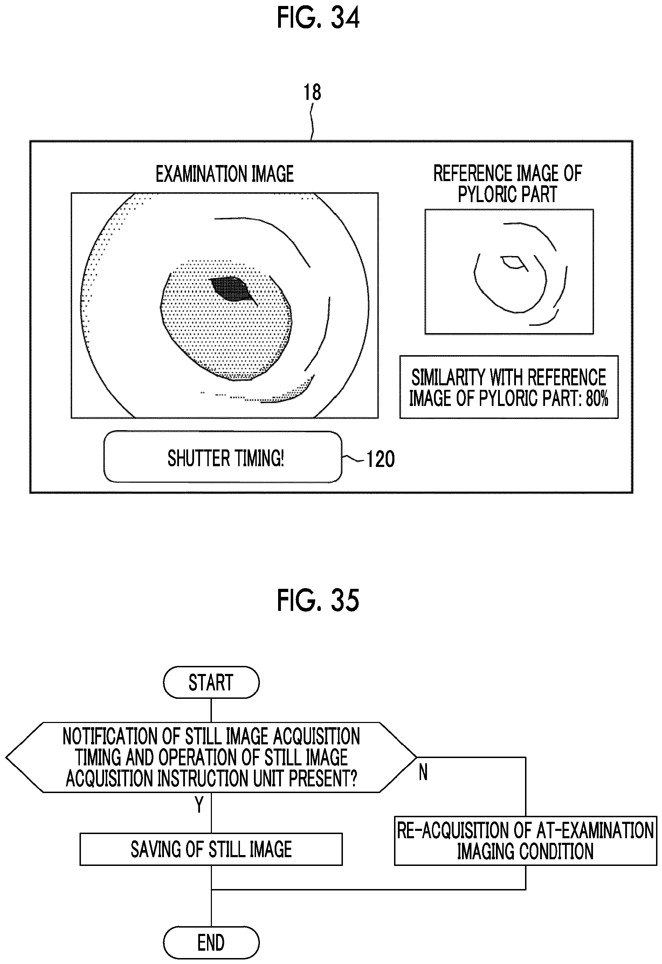

As illustrated in Fig. 34, the acquisition timing of the still image, that is, the notification of reaching the shutter timing 210 is displayed on the monitor 18 via the display control unit 54. Additionally, in a case where the notification of the acquisition timing of the still image is performed, similar to the third embodiment, the reference image selection unit 74 selects a reference image corresponding to a reference imaging condition that satisfies the at-examination imaging condition. Then, this selected reference image (the "reference image of the pyloric part" in Fig. 34) is displayed on the monitor 18. Additionally, the similarity calculation unit 76 calculates a similarity between the reference image selected by the reference image selection unit 74 and the examination image. This calculated similarity (the "similarity (80%) with the reference image of the pyloric part" in Fig. 34) is also displayed on the monitor 18. In addition, the still image acquisition instruction unit 13b in the operating part 12b may be configured of a button with a built-in light emitting diode (LED) or the like, and the notification of the acquisition timing of the still image may be performed by turning on the LED built in the still image acquisition instruction unit 13b at the acquisition timing of the still image.

As illustrated in Fig. 35, the still image storage control unit 55 performs the control of storing the still image of the examination image into the saving-image storage unit 56 as in the third embodiment in a case where the notification of the acquisition timing of the still image is performed, and the user determines that the still image of the examination image displayed on the monitor 18 is an image (reference-equivalent image) according to the guidelines and performs the operation of the still image acquisition instruction unit 13b. In addition, in a case where the still image of the examination image is saved, it is preferable to display an examination region where the still image is saved on the examination target map 90, as in the third embodiment (refer to Fig. 30).

On the other hand, the still image storage control unit 55 does not perform the saving of the still image of the examination image into the saving-image storage unit 56 in a case where the notification of the acquisition timing of the still image is performed, but the user determines that the still image of the examination image displayed on the monitor 18 is not the image (reference-equivalent image) according to the guidelines and does not perform the operation of the still image acquisition instruction unit 13b within a given time from the notification (in addition, in a case where the examination image rejection button illustrated in the third embodiment is operated). In this case, the user operates the endoscope 12 such that the endoscope 12 is located in an examination site defined by the guidelines, and performs re-acquisition of the at-examination imaging condition. In addition, in a case where the still image is not performed, as in the third embodiment, it is preferable that the operation assist information 82 required for the imaging of an image (reference-equivalent image) according to the guidelines is displayed on the monitor 18 (refer to Fig. 31).

In the above-described embodiment, hardware structures of processing units, which execute various kinds of processing, such as the image processing unit 52, are various processors as illustrated below. Various processors include a central processing unit (CPU) that is a general-purpose processor that executes software (programs) to function as various processing units, an exclusive electric circuit that is a processor having a circuit configuration exclusively designed to execute various kinds of processing, such as a programmable logic device (PLD) that is a processor capable of changing a circuit configuration after manufacture of a field programmable gate array (FPGA) or the like, and the like.

One processing unit may be configured of one of these various processors, or may be configured of a combination of two or more same or different processors (for example, a combination of a plurality of the FPGAs or the CPU and the FPGA). Additionally, the plurality of processing units may be configured of one processor. As an example in which the plurality of processing units are configured of the one processor, firstly, as represented by a computer, such as a client or a server, there is a form in which one processor is configured of a combination of one or more CPUs and software and this processor functions as the plurality of processing units. Secondly, as represented by a system-on-chip (SOC) or the like, there is a form in which a processor, which realizes functions of an overall system including the plurality of processing units with one integrated circuit (IC) chip, is used. In this way, the various processing units are configured by using one or more of the above various processors as the hardware structures.

Moreover, the hardware structures of these various processors are more specifically circuitries having a form in which circuit elements, such as semiconductor elements, are combined together.

### Explanation of References

- 10:: endoscope system
- 12:: endoscope
- 12a:: insertion part
- 12b:: operating part
- 12c:: bending part
- 12d:: distal end part
- 13a:: angle knob
- 13b:: still image acquisition instruction unit
- 13c:: mode switching unit
- 13d:: zooming operating unit
- 14:: light source device
- 16:: processor device
- 18:: monitor
- 19:: console
- 20:: light source unit
- 22:: light source control unit
- 24:: light guide
- 30a:: illumination optical system
- 30b:: imaging optical system
- 32:: imaging sensor
- 42:: detecting sensor
- 50:: image signal acquisition unit
- 52:: image processing unit
- 54:: display control unit
- 55:: still image storage control unit
- 56:: saving-image storage unit
- 57:: endoscope positional information calculation unit
- 58:: endoscope operation information calculation unit
- 60:: ordinary light mode processing unit
- 62:: special light mode processing unit
- 64:: examination assist mode processing unit
- 70:: examination assist mode image processing unit
- 72:: defocusing/blurring determination unit
- 73:: defective region removal unit
- 74:: reference image selection unit
- 74a:: reference image storage unit
- 76:: similarity calculation unit
- 78:: examination assist image producing unit
- 80:: warning display
- 82:: operation assist information
- 84:: endoscope operation information
- 86:: endoscope positional information
- 90:: examination target map
- 100:: automatic imaging mode processing unit
- 102:: automatic imaging mode image processing unit
- 104:: at-examination imaging condition acquisition unit
- 106:: imaging condition acquisition unit
- 106a:: reference imaging condition storage unit
- 108:: still image saving request signal output unit
- 120:: confirmation message
- 122:: saving completion message
- 200:: imaging assist mode processing unit
- 202:: imaging assist mode image processing unit
- 204:: still image acquisition timing notification unit
- 210:: notification of shutter timing

## Claims

1. An endoscope system (10) comprising:
an imaging condition determination unit including a reference imaging condition storage unit (106a) that stores a reference imaging condition for obtaining a predetermined reference image for each examination region;
the endoscope system further comprising:
an image acquisition unit (50) that images the examination region to acquire an examination image in order to obtain a reference-equivalent image equivalent to the reference image;
an at-examination imaging condition acquisition unit (104) that acquires an at-examination imaging condition obtained at an acquisition timing of the examination image;
a still image saving request signal output unit (108) that outputs a still image saving request signal for requesting saving of a still image of the examination image; and
a display unit (18) that displays the examination image, **characterized in that**:
the imaging condition determination unit is configured to determine whether or not the at-examination imaging condition satisfies the reference imaging condition for obtaining a reference image, such that in a case where the at-examination imaging condition satisfies the reference imaging condition the still image saving request signal output unit outputs a still image saving request signal, which requests the saving of the still image of the examination image.

2. The endoscope system according to claim 1, further comprising:
a reference image storage unit (74a) that stores a plurality of the reference images; and
a reference image selection unit (74) that selects a reference image corresponding to the examination image from the reference image storage unit,
wherein the display unit (18) displays the reference image selected by the reference image selection unit in a case where the still image saving request signal is output.

3. The endoscope system according to claim 1, further comprising:
a reference image storage unit (74a) that stores a plurality of the reference images;
a reference image selection unit (74) that selects a reference image corresponding to the examination image from the reference image storage unit; and
a similarity calculation unit (76) that calculates a similarity between the reference image selected by the reference image selection unit and the examination image,
wherein the display unit (18) displays the similarity in a case where the still image saving request signal is output.

4. The endoscope system according to any one of claims 1 to 3, further comprising: a still image storage control unit (55) that performs a control of storing the still image of the examination image into a saving-image storage unit in a case where the still image saving request signal is output and operation of a still image acquisition instruction unit is performed.

5. The endoscope system according to claim 4,
wherein in a case where the still image of the examination image is obtained in a specific examination region, the display unit (18) displays that the still image of the examination image in the specific examination region has been saved in the saving-image storage unit (56) on an examination target map.

6. The endoscope system according to any one of claims 1 to 5,
wherein the display unit (18) displays operation assist information on the operation of an endoscope (12) in a case where the still image saving request signal is output and the still image of the examination image is not stored in a saving-image storage unit.

7. The endoscope system according to any one of claims 1 to 6, further comprising:
a mode switching unit (13c) that performs switching to an automatic imaging mode in which the still image of the examination image is automatically acquired in a case where the at-examination imaging condition satisfies the reference imaging condition.

8. The endoscope system according to any one of claims 1 to 7,
wherein the reference imaging condition is at least one of endoscope positional information or endoscope operation information for obtaining the reference image, and the at-examination imaging condition is at least one of endoscope positional information or endoscope operation information for obtaining the examination image.

9. The endoscope system according to claim 8,
wherein the endoscope operation information includes at least one of a bending angle or rotational amount of an endoscope, a distance from an observation object, an insertion length of an insertion part of the endoscope (12), and a shape of the insertion part of the endoscope.

## Patentansprüche

1. Endoskopsystem (10) umfassend:
eine Bildgebungsbedingungs-Bestimmungseinheit mit einer Referenz-Bildgebungsbedingungs-Speichereinheit (106a), die eine Referenz-Bildgebungsbedingung zum Erhalten eines vorbestimmten Referenzbilds für jede Untersuchungszone speichert; wobei das Endoskopsystem weiterhin aufweist:
eine Bilderfassungseinheit (50), die die Untersuchungszone abbildet, um ein Untersuchungsbild zu erfassen und ein zu dem Referenzbild äquivalentes Referenz-Äquivalenzbild zu gewinnen;
eine Untersuchungs-Bildgebungsbedingungs-Erfassungseinheit (104), die eine Bildgebungsbedingung bei der Untersuchung erfasst, gewonnen zu einer Erfassungszeit des Untersuchungsbilds;
eine Stehbild-Speicheranforderungssignal-Ausgabeeinheit (108), die ein Stehbild-Speicheranforderungssignal zum Anfordern der Speicherung eines Stehbilds des Untersuchungsbilds ausgibt;
eine Anzeigeeinheit (18), die das Anzeigebild anzeigt, **dadurch gekennzeichnet, dass**
die Bildgebungsbedingungs-Bestimmungseinheit konfiguriert ist zum Bestimmen, ob die Untersuchungs-Bildgebungsbedingung die Referenz-Bildgebungsbedingung zum Erhalt eines Referenzbilds erfüllt oder nicht, sodass dann, wenn die Untersuchungs-Bildgebungsbedingung die Referenz-Bildgebungsbedingung erfüllt, die Stehbild-Speicheranforderungssignal-Ausgabeeinheit ein Stehbild-Speicheranforderungssignal ausgibt, welches das Speichern des Stehbilds des Untersuchungsbilds anfordert.

2. Endoskopsystem nach Anspruch 1, weiterhin umfassend:
eine Referenzbild-Speichereinheit (74a), die eine Mehrzahl der Referenzbilder speichert; und
eine Referenzbild-Auswahleinheit (74), die ein Referenzbild entsprechend dem Untersuchungsbild aus der Referenzbild-Speichereinheit auswählt,
wobei die Anzeigeeinheit (18) das von der Referenzbild-Auswahleinheit ausgewählte Referenzbild dann anzeigt, wenn das Stehbild-Speicheranforderungssignal ausgegeben wird.

3. Endoskopsystem nach Anspruch 1, weiterhin umfassend:
eine Referenzbild-Speichereinheit (74a), die eine Mehrzahl der Referenzbilder speichert;
eine Referenzbild-Auswahleinheit (74), die ein Referenzbild entsprechend dem Untersuchungsbild aus der Referenzbild-Speichereinheit auswählt; und
eine Ähnlichkeitsberechnungseinheit (76), die eine Ähnlichkeit zwischen dem von der Referenzbild-Auswahleinheit ausgewählten Referenzbild und dem Untersuchungsbild berechnet, wobei die Anzeigeeinheit (18) die Ähnlichkeit gern anzeigt, wenn das Stehbild-Speicheranforderungssignal ausgegeben wird.

4. Endoskopsystem nach einem der Ansprüche 1 bis 3, weiterhin umfassend:
eine Stehbild-Speichersteuereinheit (55), die eine Steuerung des Speicherns des Stehbilds des Untersuchungsbilds in einer Sicherungsbild-Speichereinheit dann ausführt, wenn das Stehbild-Speicheranforderungssignal ausgegeben wird und der Betrieb einer Stehbilderfassungs-Befehlseinheit ausgeführt wird.

5. Endoskopsystem nach Anspruch 4,
bei dem dann, wenn das Stehbild des Untersuchungsbilds in einer spezifischen Untersuchungszone erhalten wird, die Anzeigeeinheit (18) anzeigt, dass das Stehbild des Untersuchungsbilds in der spezifischen Untersuchungszone in der Sicherungs-Bildspeichereinheit (56) auf einer Untersuchungszielkarte gesichert wurde.

6. Endoskopsystem nach einem der Ansprüche 1 bis 5,
bei dem die Anzeigeeinheit (18) Bedienungs-Unterstüzungsinformationen für die Bedienung eines Endoskops (12) dann anzeigt, wenn das Stehbild-Speicheranforderungssignal ausgegeben wird und das Stehbild des Untersuchungsbilds nicht in einer Sicherungs-Bildspeichereinheit gesichert ist.

7. Endoskopsystem nach einem der Ansprüche 1 bis 6, weiterhin umfassend:
eine Modus-Umschalteinheit (13c), die eine Umschaltung in einen automatischen Bildgebungsmodus, in welchem das Stehbild des Untersuchungsbilds automatisch erfasst wird, dann ausführt, wenn die Untersuchungs-Bildgebungsbedingung die Referenz-Bildgebungsbedingung erfüllt.

8. Endoskopsystem nach einem der Ansprüche 1 bis 7,
bei dem die Referenz-Bildgebungsbedingung Endoskoplageinformationen und/oder Endoskopbedieninformationen zum Erhalten des Referenzbilds ist, und die Untersuchungs-Bildgebungsbedingung Endoskoplageinformationen und/oder Endoskopbedienungsinformationen zum Erhalten des Untersuchungsbilds ist.

9. Endoskopsystem nach Anspruch 8,
bei dem die Endoskopbedienungsinformation einen Biegewinkel einen Drehbetrag eines Endoskops, eine Entfernung von einem Betrachtungsobjekt, eine Einführlänge eines Einführteils des Endoskops (12) und/oder eine Form eines Einführteils des Endoskops enthält.

## Revendications

1. Système d'endoscope (10), comprenant :
une unité de détermination de condition d'imagerie incluant une unité de stockage de condition d'imagerie de référence (106a), laquelle stocke une condition d'imagerie de référence pour obtenir une image de référence prédéterminée pour chaque région d'examen, le système d'endoscope comprenant en outre :
une unité d'acquisition d'image (50), laquelle image la région d'examen pour acquérir une image d'examen afin d'obtenir une image équivalente de référence, équivalant à l'image de référence ;
une unité d'acquisition de condition d'imagerie lors de l'examen (104), laquelle acquiert une condition d'imagerie lors de l'examen obtenue lors de la temporisation d'acquisition de l'image d'examen ;
une unité de sortie de signal de demande de sauvegarde d'image fixe (108), laquelle produit un signal de demande de sauvegarde d'image fixe pour demander la sauvegarde d'une image fixe de l'image d'examen, et
une unité d'affichage (18), laquelle affiche l'image d'examen,
**caractérisée en ce que** :
l'unité de détermination de condition d'imagerie est configurée pour déterminer si oui ou non la condition d'imagerie lors de l'examen satisfait la condition d'imagerie de référence pour obtenir une image de référence, de telle sorte que dans un cas où la condition d'imagerie lors de l'examen satisfait la condition d'imagerie de référence, l'unité de sortie de signal de demande de sauvegarde d'image fixe produit un signal de demande de sauvegarde d'image fixe, lequel demande la sauvegarde de l'image fixe de l'image d'examen.

2. Système d'endoscope selon la revendication 1, comprenant en outre :
une unité de stockage d'image de référence (74a), laquelle stocke une pluralité des images de référence, et
une unité de sélection d'image de référence (74), laquelle sélectionne une image de référence correspondant à l'image d'examen dans l'unité de stockage d'image de référence ;
dans lequel l'unité d'affichage (18) affiche l'image de référence sélectionnée par l'unité de sélection d'image de référence dans un cas où le signal de demande de sauvegarde d'image fixe est produit.

3. Système d'endoscope selon la revendication 1, comprenant en outre :
une unité de stockage d'image de référence (74a), laquelle stocke une pluralité des images de référence, et
une unité de sélection d'image de référence (74), laquelle sélectionne une image de référence correspondant à l'image d'examen dans l'unité de stockage d'image de référence, et
une unité de calcul de similarité (76), laquelle calcule une similarité entre l'image de référence sélectionnée par l'unité de sélection d'image de référence et l'image d'examen,
dans lequel l'unité d'affichage (18) affiche la similarité dans un cas où le signal de demande de sauvegarde d'image fixe est produit.

4. Système d'endoscope selon l'une quelconque des revendications 1 à 3, comprenant en outre : une unité de commande de stockage d'image fixe (55), laquelle réalise une commande pour stocker l'image fixe de l'image d'examen dans une unité de stockage d'image de sauvegarde, dans un cas où le signal de demande de sauvegarde d'image fixe est produit et le fonctionnement d'une unité d'instruction d'acquisition d'image fixe est réalisé.

5. Système d'endoscope selon la revendication 4,
dans lequel dans un cas où l'image fixe de l'image d'examen est obtenue dans une région d'examen spécifique, l'unité d'affichage (18) affiche que l'image fixe de l'image d'examen dans la région d'examen spécifique a été sauvegardée dans l'unité de stockage d'image de sauvegarde (56) sur une carte cible d'examen.

6. Système d'endoscope selon l'une quelconque des revendications 1 à 5,
dans lequel l'unité d'affichage (18) affiche des informations d'assistance de fonctionnement sur le fonctionnement d'un endoscope (12) dans un cas où le signal de demande de sauvegarde d'image fixe est produit et l'image fixe de l'image d'examen n'est pas stockée dans une unité de stockage d'image de sauvegarde.

7. Système d'endoscope selon l'une quelconque des revendications 1 à 6, comprenant en outre :
une unité de commutation de mode (13c), laquelle réalise une commutation sur un mode d'imagerie automatique, où l'image fixe de l'image d'examen est automatiquement acquise dans un cas où la condition d'imagerie lors de l'examen satisfait la condition d'imagerie de référence.

8. Système d'endoscope selon l'une quelconque des revendications 1 à 7,
dans lequel la condition d'imagerie de référence est au moins une condition parmi des informations de position d'endoscope ou des informations de fonctionnement d'endoscope pour obtenir l'image de référence, et la condition d'imagerie lors de l'examen est au moins une condition parmi des informations de position d'endoscope ou des informations de fonctionnement d'endoscope pour obtenir l'image d'examen.

9. Système d'endoscope selon la revendication 8,
dans lequel les informations de fonctionnement d'endoscope incluent au moins des informations parmi un angle de flexion ou une quantité de rotation d'un endoscope, une distance à partir d'un objet d'observation, une longueur d'introduction d'une partie d'introduction de l'endoscope (12), et une forme de la partie d'introduction de l'endoscope.
